# EUROPEAN PATENT APPLICATION

(11) **EP 4 269 413 A1**
(43) Date of publication of application: **01.11.2023**
(21) Application number: 22170618.7
(22) Date of filing: 28.04.2022
(51) Int. Cl.: C07D 487/22, B01D 69/02, B01D 71/06, C01B 32/15, C07C 25/18

(54) **A METHOD FOR SYNTHESIZING A TWO-DIMENSIONAL MATERIAL CONSISTING OF CONJUGATED AROMATIC STRUCTURES BY TRIMERIZATION**

(71) Applicant: Freie Universität Berlin, 14195 Berlin (DE)
(72) Inventor: MOHSEN, Aadeli, 12209 Berlin (DE); REICH, Stephanie, 10779 Berlin (DE); SETARO, Antonio, 10715 Berlin (DE); BEYRANVAND, Siamak, Khorramabad, Lorestand (IR)
(74) Representative: Maikowski & Ninnemann Patentanwälte Partnerschaft mbB

(57) **Abstract**

The present invention relates to a method for synthesizing two-dimensional material consisting of conjugated aromatic structures by trimerization, which comprises reacting sodium acetylide or sodium cyanide, and at least one electron deficient aromatic system of the general formula (I).

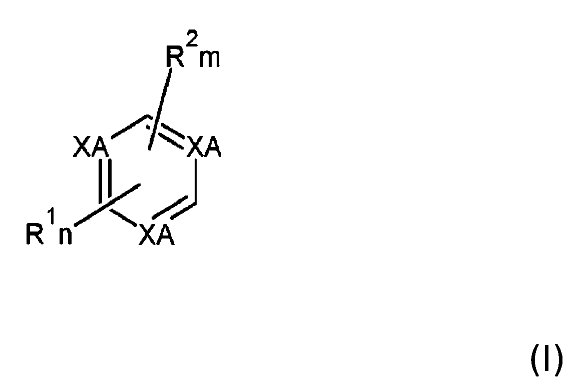

## Description

The present invention relates to a method for synthesizing a two-dimensional material consisting of conjugated aromatic structures by trimerization, the two-dimensional material obtained by said method and use of the two-dimensional material.

### Description

With the development of covalent organic frameworks and carbon nanostructures, a great deal of attention has been paid to the cost-effective carbon-carbon couplings through which multifunctional monomers are polymerized into π-conjugated networks (Hu, F.; Hao, W.; Mücke, D.; Pan, Q.; Li, Z.; Qi, H.; Zhao, Y., Highly Efficient Preparation of Single- Layer Two-Dimensional Polymer Obtained from Single-Crystal to Single-Crystal Synthesis. Journal of the American Chemical Society 2021, 143 (15), 5636-5642).

Among the carbon- carbon couplings, the cyclization of alkynes to the benzene ring has shown great promise for the construction of new materials, due to the its versatility (Klappenberger, F.; Zhang, Y.-Q.; Björk, J.; Klyatskaya, S.; Ruben, M.; Barth, J. V., On-Surface Synthesis of Carbon-Based Scaffolds and Nanomaterials Using Terminal Alkynes. Accounts of Chemical Research 2015, 48 (7), 2140-2150).

This reaction can be mediated by alkene, e.g Bergman cyclization (Schuler, B.; Fatayer, S.; Mohn, F.; Moll, N.; Pavliček, N.; Meyer, G.; Peña, D.; Gross, L., Reversible Bergman cyclization by atomic manipulation. Nature Chemistry2016, 8 (3), 220-224), or without any secondary unit, the so called trimerization.

Cyclization of alkynes is usually catalyzed by transition-metal complexes and is a very powerful tool in the arsenal of organic chemists for the construction of benzene derivatives in a one-pot reaction. In this reaction each alkyne molecule form two new carbon-carbon σ bonds at the expense of one π bond. Over several decades the mechanism of this reaction has been intensively studied (Galan, B. R.; Rovis, T., Beyond Reppe: Building Substituted Arenes by [2+2+2] Cycloadditions of Alkynes. Angewandte Chemie International Edition 2009, 48 (16), 2830-2834).

As the core area of interest, two steps including coordination of catalyst with the π bonds of two alkyne units and cyclization of the created metallacyclopentadienes with a further alkyne through intramolecular [4+2] cycloaddition or Schore route have been verified for the catalyst action.

Great efforts have been devoted to develop different catalysts and to optimize the yield, chemo-, stereo- and regioselectivity of this reaction (Lipschutz, M. I.; Chantarojsiri, T.; Dong, Y.; Tilley, T. D., Synthesis, Characterization, and Alkyne Trimerization Catalysis of a Heteroleptic Two-Coordinate Fel Complex. Journal of the American Chemical Society 2015, 137(19), 6366-6372).

While understanding of catalyst action opened new ways for regulating cyclotrimerization of alkynes, other important parameters such as the structural effects of the reagents have been almost ignored. Therefore, the development of [2+2+2] cycloaddition to the synthesis of advanced materials, such as carbon nanostructures, has been limited by catalyst efficiency and any variation in the catalyst action induces a high discrepancy in the long range structure of the product. For example, physical and chemical interactions between the backbone of the carbon platforms and the catalyst increase the dispersity of the product and affect the reproducibility of the reaction. Due to such problems, studying the mechanism of the long-range cyclotrimerization towards carbon nanostructures and optimizing the reaction's key factors remains challenging.

As an alternative, the catalyst-free cyclotrimerization of alkynes under various conditions has been studied and valuable results have been achieved Hapke, M., Transition metal-free formal [2+2+2] cycloaddition reactions of alkynes. Tetrahedron Letters 2016, 57 (51), 5719-5729). Regardless of the required co-catalysts and co-reagents, the catalyst-free cyclotrimerization of alkynylketones at relatively low temperatures promised new pathways for the cyclization of alkynes, circumventing the catalyst- dependent limitations and side reactions at high temperatures. The polarization of carbon-carbon triple bonds by adjacent carbonyl groups plays a crucial role in their susceptibility to trimerization. This effect is particularly important for the long range carbon-carbon couplings and preparation of large carbon nanostructures, as catalyst limitations and backbone destruction of carbon nanostructures at high temperatures can be circumvented.

However, it has been shown that the use of alkynylketones does not support the synthesis of larger sheet like aromatic structures.

Thus, it was an object of the present invention to provide a method that enables the synthesis of sheet like aromatic structures with lateral dimensions in the range of several micrometers.

This object has been solved by providing a method with the features of claim 1 and a sheet-like material as defined in claims 9 and 10.

Accordingly, a method for synthesizing a two-dimensional, sheet-like material consisting of conjugated aromatic structures by trimerizationis provided, wherein the method comprises reacting
- sodium acetylide or cyanides, in particular sodium acetylide; and
- at least one electron deficient aromatic system of the general formulae (I) 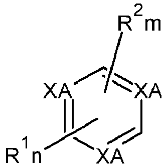
   with R¹ being an electron withdrawing moiety selected from halogen, in particular
   -Cl or -F, -CF₃, -OCF₃, B(ORa)₂, SiRa₃, Si(ORa)₃ , with Ra being H or alkyl, in particular C1-C10 alkyl,
   with n being 1-6,
   with R² being selected from C1-C6 alkyl, C1-C6 alkoxy or substituted or unsubstituted C6 aryl moiety,
   with m being 0-5,
   with XA being independently from each other N, S, O or CH.

The method according to the invention allows for an electron deficient catalyst-free trimerization of alkynes and cyanides or indeed any molecule having a triple bond at room temperature for the construction of a wide range of organic frameworks with conjugated aromatic backbone.

The method according to the invention is a new approach for the synthesis of a wide range of organic frameworks exemplified by sheet-like conjugated aromatic structures, such as benzene-triazine heteroaromatic structures with lateral dimensions in the range of several micrometers by the catalyst free cyclotrimerization of alkynes at room temperature. The sheet-like benzene-triazine heteroaromatic structures are synthesized by a cost-effective and straightforward procedure including one-pot catalyst free cyclotrimerization of alkynes at room temperature.

The attachment of ethynyl group to an electron deficient aromatic ring, such as cyanuric chloride, resulted in highly reactive intermediates that transform to sheet-like structures comprising benzene and another aromatic rings upon cyclotrimerization at ambient conditions. The reaction can be performed on gram scale without using any catalyst or co-catalyst. Experimental data showed that an electron poor segment, such as traizine, increased the reactivity of ethynyl groups toward nucleophiles and cyclotrimerization.

It has also been shown, that the sheet-like structures obtained by this method can be used for molecular storage and filtering applications. While small molecules, such as rhodamine 6g were able to penetrate into sheet-like benzene-triazine heteroaromatic structures intensively, larger objects such as 5 nm gold nanoparticles were not able to pass through the layers. Thus, the synthetic method according to the invention can be used for the production of a wide range of aromatic and heteroaromatic structures by manipulation of the structure of ethynyl and electron deficient segments.

In an embodiment of the present method R¹ is an electron withdrawing moiety selected from -Cl or -F, with n being 1-6, and R² is selected from C1-C4 alkyl, such as -CH₃, -C2H₅, - C₃H₇, C1-C4 alkoxy, such as -OCH₃, -OC₂H₅ or -OC₃H₇, or phenyl, with m being 0-5, and with XA being independently from each other N or CH.

In a further embodiment of the present method, the at least one electron deficient aromatic system is one of the general formulae (IIa-c) 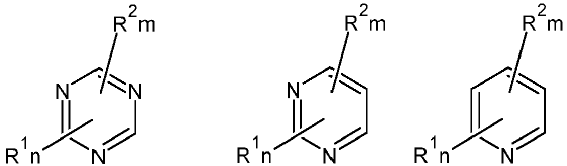
with R¹ being an electron withdrawing moiety selected from halogen, in particular -Cl or-F, -CF₃, -OCF₃,
with n being 1, 2, 3, 4 or 5, in particular 1, 2 or 3.
with R² being selected from C1-C4 alkyl, such as -CH₃, -C2H₅, - C₃H₇, C₁-C₄ alkoxy, such as OCH₃, -OC₂H₅ or -OC₃H₇, or phenyl,
with m being 0, 1, 2, 3 or 4.

In yet another embodiment of the present method, the at least one electron deficient aromatic system is one of the general formulae (IIIa-c) 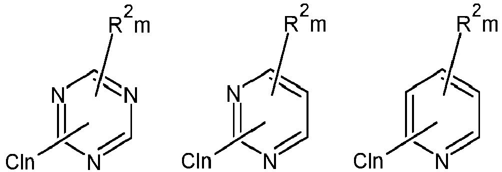
with n being 1, 2, 3, 4 or 5, in particular 1, 2 or 3,
with R² being selected from C1-C4 alkyl, such as -CH₃, -C2H₅, - C₃H₇, C₁-C₄ alkoxy, such as OCH₃, -OC₂H₅ or -OC₃H₇, or phenyl,
with m being 0, 1, 2, 3 or 4.

In still another preferred embodiment of the present method, the at least one electron deficient aromatic system is one of the following triazines: 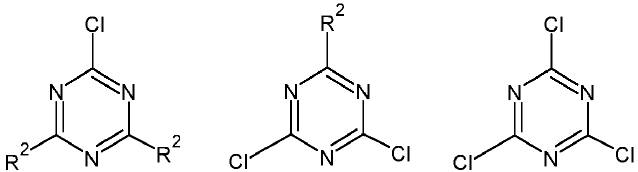 with R² being selected from C1-C4 alkyl, such as -CH₃, -C2H₅, - C₃H₇, C₁-C₄ alkoxy, such as OCH₃, -OC₂H₅ or -OC₃H₇, or phenyl.

Specific compounds with an electron deficient aromatic systems may be one of the following triazines: 2,4,6-trichloro-1,3,5-trazine (cyanuric chloride), 2-chloro-4,6-diphenyl-1,3,5-triazine, 2-chloro-4,6-methoxy-1,3,5-triazine, 2,4-dichloro-6-methoxy-1,3,5-traizine.

In another embodiment of the present method, the at least one electron deficient aromatic system may be of the general formulae (IV) 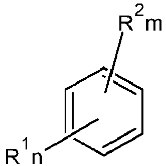
with R¹ being an electron withdrawing moiety selected from halogen, in particular -Cl or-F, -CF₃, -OCF₃,
with n being 1-6, in particular 4, 5 or 6,
with R² being selected from C1-C4 alkyl, such as -CH₃, -C2H₅, - C₃H₇, C₁-C₄ alkoxy, such as OCH₃, -OC₂H₅ or -OC₃H₇, or phenyl,
with m being 0, 1, 2, 3 , 4 or 5, in particular 0, 1, 2.

In a further embodiment of the present method, the at least one electron deficient aromatic system may be of the general formulae (V) 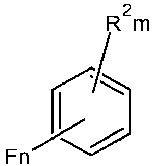
with n being 1-6, in particular 4, 5 or 6,
with R² being selected from C1-C4 alkyl, such as -CH₃, -C2H₅, - C₃H₇, C₁-C₄ alkoxy, such as OCH₃, -OC₂H₅ or -OC₃H₇, or phenyl,
with m being 0, 1, 2, 3 , 4 or 5, in particular 0, 1, 2.

In still a further embodiment of the present method, the at least one electron deficient aromatic system is one of the following: 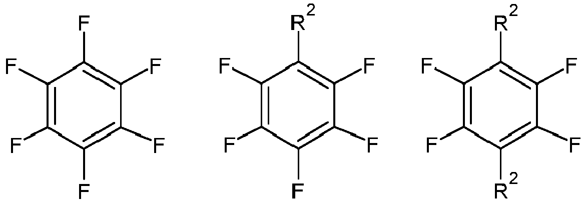 with R² being selected from C1-C4 alkyl, such as -CH₃, -C2H₅, - C₃H₇, C₁-C₄ alkoxy, such as OCH₃, -OC₂H₅ or -OC₃H₇, or phenyl.

A preferred compound is perfluorated benzene (F₆-benzene).

In one further embodiment of the present method at least two of the electron deficient aromatic system of the general formulae (I) are reacted with sodium acetylide. For example at least two of the following triazine compounds may be reacted with sodium acetylide: 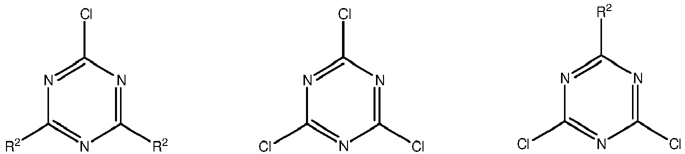 with R² being selected from C1-C4 alkyl, such as -CH₃, -C₂H₅, - C₃H₇, C₁-C₄ alkoxy, such as OCH₃, -OC₂H₅ or -OC₃H₇, or phenyl.

The present method is carried out in the presence of an organic solvent, in particular THF, at room temperature.

As mentioned above, the present method enables the synthesis of a two-dimensional material with conjugated aromatic structures, comprising at least one of the following structure units in a first aspect: 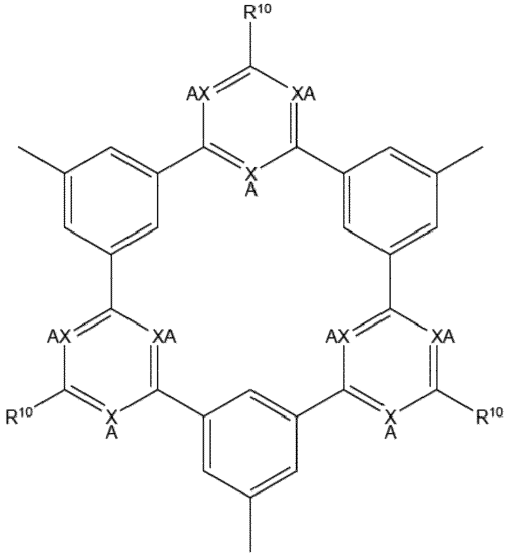
with XA being independently from each other N, S, O or CH, in particular N or CH;
with R¹⁰ being selected from -CHCH, -OH, C1-C6 alkyl, in particular C1-C4 alkyl, such as -CH₃, -C2H₅, - C₃H₇, C1-C6 alkoxy, in particular C₁-C₄ alkoxy, such as -OCH₃, -OC₂H₅ or -OC₃H₇, substituted or unsubstituted C6 aryl moiety , in particular phenyl.

In another aspect, a two-dimensional material with conjugated aromatic structures obtainable in the method as described comprises at least one of the following structure units: 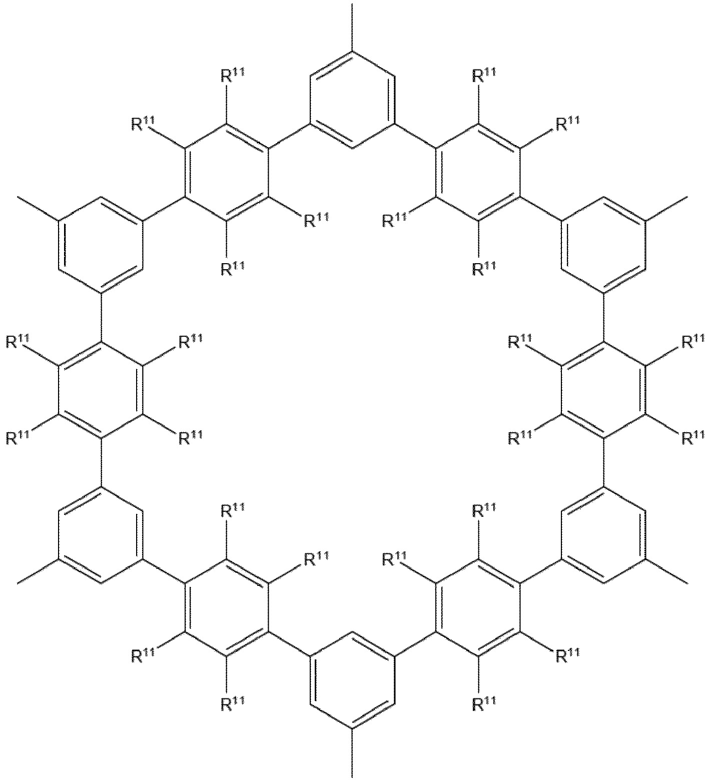 with R¹¹ being an electron withdrawing moiety selected from halogen, in particular -Cl or -F, -CF₃, -OCF₃, B(ORa)₂ , SiRa₃, Si(ORa)₃with Ra being H or alkyl, in particular C1-C10 alkyl, most preferably -F.

It is to be understood that the illustrated structure units represent only one section or selection of the sheet-like two-dimensional material obtained by the present method.

The growth of the sheets depends on the reaction time. For example, a product of a 2 hours reaction consisted of inhomogeneous flat materials laterally connected with bigger sheets. After six hours, sheet-like structures with clear edges in the micrometer range were formed. Small particles joining the edges of the big sheets indicated sheets with lateral growth. After 18 hours reaction, the product contained sheet-like structures with several micrometers lateral size and clear edges.

In one embodiment, the sheet like material has the following structure:

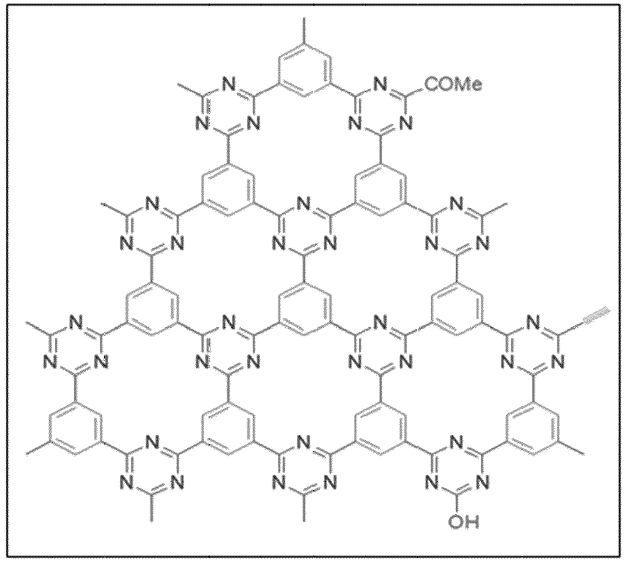

In a further embodiment, the sheet like material has the following structure:

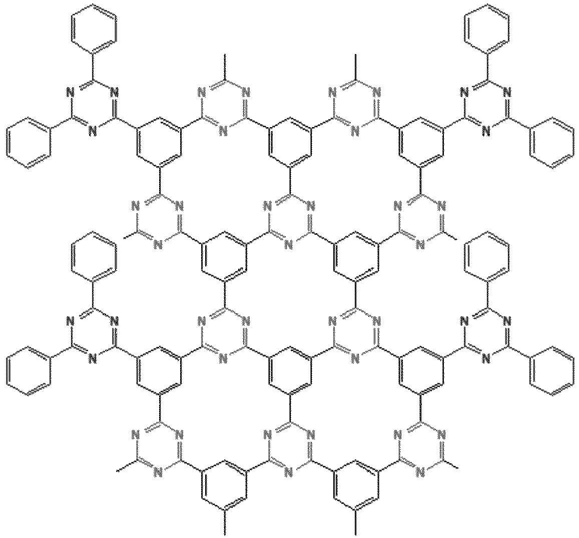

In still another embodiment, the sheet like material has the following structure:

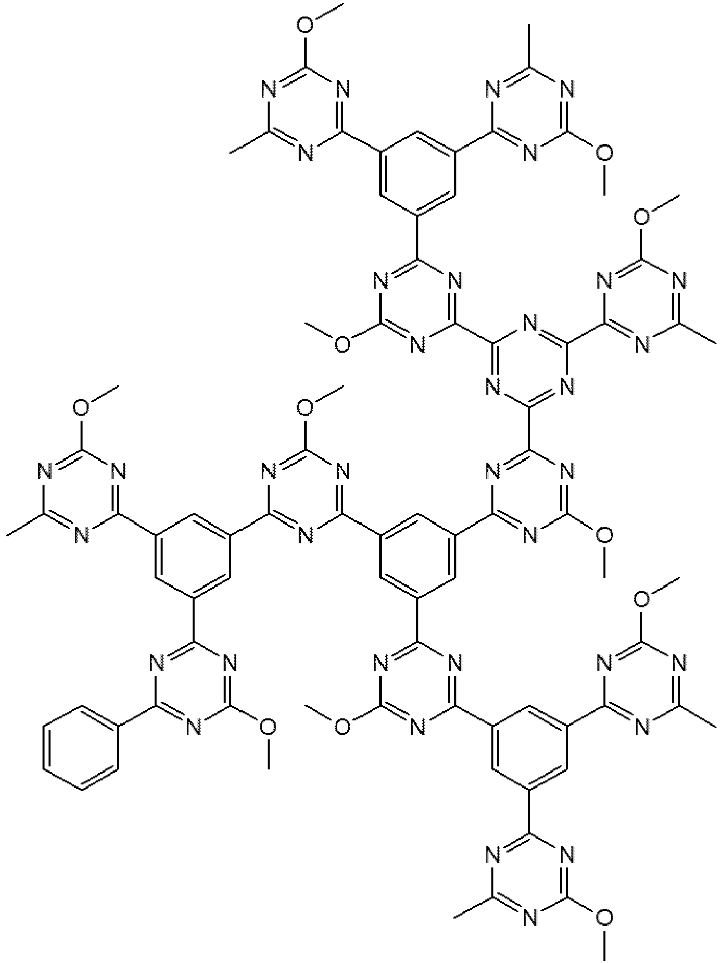

In still a further embodiment, the sheet like material has one of the following structures: 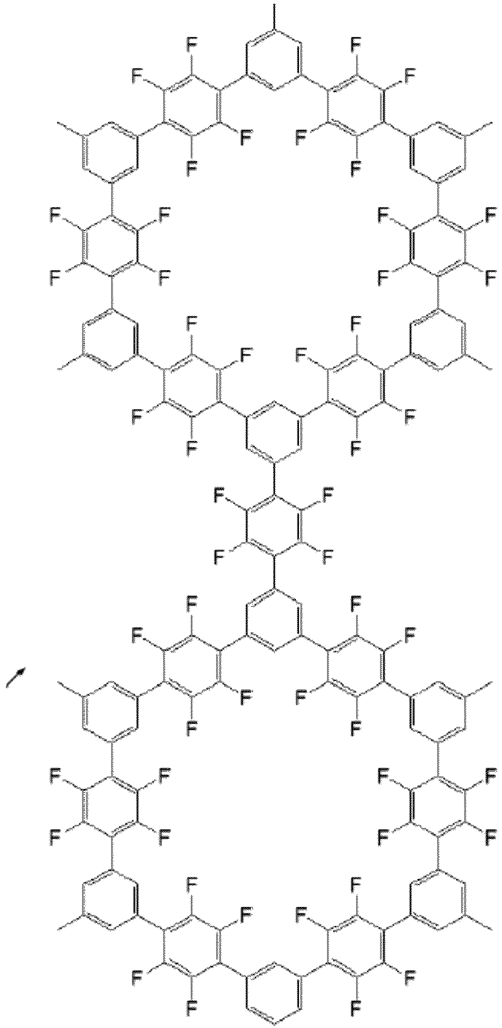 or

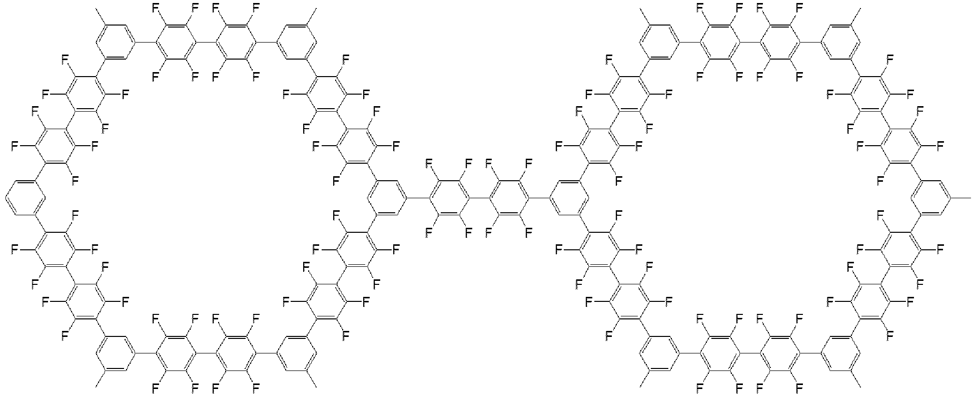

The two-dimensional material as described may be used as filter material for small molecules or compounds, in particular molecules with a size larger 5 nm.

The invention is explained in the following by means of the examples with reference to the figures. It shows:
- FIG 1A: a schematic representation of the synthesis of sheet-like polytriazine heteroaromatic structures;
- FIG 1B: Atomic force microscopy (AFM) and conventional transmission electron microscopy (TEM) images of the product of reactions at 2h, 6h and 18 h. (e) Scanning electron microscopy (SEM) images with a low (up) and high (bottom) magnifications of sheet-like polytriazine
- FIG 2A: Schematic representation of lateral growing and vertical stacking of PTrz sheets.
- FIG 2B: AFM image of an exfoliated sheet of PTrz18.
- FIG 2C: Cryo-TEM image of PTrz18 in water, displaying several sheets with a flat morphology.
- FIG 2D: 13C CP-MAS-NMR spectra of PTrz showed distinguished signals for the triazine and benzene rings as well as ethynyl and methyl ketone functional groups.
- FIG 2E: Raman spectra of molecular models
- FIG 2F: with a structure close to the repeating unit of PTrz.
- FIG 2G: Raman spectra of few layer PTrz18, showing significant changes in vibrational modes upon exfoliation.
- FIG 2H: Composition of PTrz2, PTrz6 and PTrz18 based on elemental analysis.
- FIG 2I: TGA themograms of PTrz2, PTrz6 and PTrz18 at a heating rate of 10 °C/min under argon.
- FIG 3A: Schematic representation of intercalation of rhodamine 6g by nonexfoliated PTrz18. The porous structure of layers of PTrz18 allowed dye molecules to penetrate deep in this compound.
- FIG 3B: UV-vis spectra of PTrz, free rhodamine 6g and PTrz/rh in water solution.
- FIG 3C: PLE maps of different concentrations of free rhodamine 6g and PTrz/rh in water.
- FIG 3D: Luminescence of free rhodamine 6g and PTrz/rh in the solid state. A water solution of samples with 0.1 µg/ml concentration was dropped on silica surface and dried under argon and excited by laser 532 nm;
- FIG 4A: Schematic representation of interaction between gold nanoparticles and PTrz18. Beacuse the size of the gold nanoparticles is larger than the pore size of PTrz18, they cannot pass layers efficiently.
- FIG 4B: Cryo-ET of PTrz18 with 5 nm AuNP: On the left the reconstruction of the 3D structure calculated from tilt series (-64° to +64°, increment 2°) is shown (scale bar corresponds to 100 nm). On the right, the 3D volume is rotated 45° around the y-axis. The colored frames indicate orthoslices through the 3D volume at a distance of about 23 nm each (cp. the enlarged section).
- FIG 4C: The orthoslices in planar view. The red arrows mark the occurrence of single 5 nm gold nanoparticles in each slice. Only very few particles are present and only at the edges of the sheets or within defects.
- FIG 4D: Enlarged section of the arrows and the gold nanoparticles. Single slices in full resolution can be found
- FIG 5: a schematic representation of the synthesis of sheet-like polyaromatic structures containing F moieties

### Materials

2,4,6-trichloro-1,3,5-triazine (cyanuric chloride or triazine), sodium acetylide (18 wt. % slurry in xylene), biphenyl, 2-chloro-4 6-diphenyl-1 3 5-triazine, N-Methyl-2-pyrrolidone (NMP), gold nanoparticles (5 nm) and dialysis tube (benzylated tube MWCO: 50000) were purchased from Sigma-Aldrich (Schnelldorf, Germany). Dimethylformamide (DMF), toluene, mesitylene, methanol, ethanol, chloroform, acetone, tetrahydrofuran and rhodamine 6G were purchased from Merck (Germany).

### Methods

Fourier transform infrared spectroscopy (FTIR): IR spectra were recorded at room temperature using a Shimadzu FT-IR 8400 spectrometer with a KBr pellet (weight ratio 5/200 mg) at 4000 cm-1 to 500 cm-1 scanning range.

Ultraviolet-Visible spectroscopy (UV-Vis): Ultraviolet-visible-infrared absorption spectra were recorded using a Perkin-Elmer Lambda 950 spectrophotometer at room temperature. Fluorescence spectroscopy: Fluorescence maps of molecular probes were obtained by a Nanolog spectrofluorometer from Horiba, equipped with a Xenon lamp. Single excitation lines from HgXe short arc lamp were selected using a monochromator. A thermoelectrically cooled CCD detector was used to record the molecular spectra.

Fluorescence spectroscopy in solid state: For measuring fluorescence the same Horiba XploRA setup as for Raman was used, a different grating with 600 lines/mm provided high spectral range and the final spectrum were stitched together out multiple acquisitions by varying central position of the grating.

Thermal gravimetric analysis (TGA): The TGA measurements were recorded by a STA 409 apparatus (Linseis) at temperatures range 25-800 ºC with a 10 ºC/min heating rate under air. Elemental analysis (EA): EA was performed using ELEMENTAR apparatus with three columns and detector for carbon, nitrogen, and hydrogen elements.

Nuclear magnetic resonance spectroscopy (NMR): 1H-NMR and 13C-NMR spectra were recorded on a Jeol ECX 400 spectrometer or on a Bruker BioSpin Avance 700 spectrometer (Bruker Corporation, Billerica, MA, USA) (at 295 K). Chemical shifts were reported in ppm using the deuterated solvent peak as the internal standard and tetramethyl silane (SiMe4) was used for internal calibration at 125 MHz with complete proton decoupling.

13C Solid-state NMR (cross polarization magic-angle spinning (CP/MAS)): SNMR Spectra were obtained using a JEOL ECZ600 MHz spectrometer operating at 150.9 MHz.

UHPLC-ESI-IMS-MS/MS: UHPLC-ESI-IMS-MS/MS equipped with a DAD detector and a standard separation column (RP, C18) was used to measure the mass of our molecular agents. We acquired the melting points of charge transfer agents at a ramp rate of 5°C/min using Stuart Melting Point SMP30.

Matrix-Assisted Laser Desorption/Ionization Time-of-Flight Mass Spectroscopy (MALDI- TOF): Spectra were recorded using a Bruker Ultra-flex TOF/TOF in the positive ion mode using a linear pathway (LP). Alpha-cyano-4-hydroxycinnamic acid was used as the matrix. The samples were prepared via the "dried-droplet method" by dropping 0.5 µL of methanolic solution of the sample mixture and letting them dry in air. The mixture was prepared by mixing 100 µl of a 2 mg.ml-1 solution of the sample with 100 µl of a saturated matrix solution. Scanning force microscopy (SFM): Scanning force microscopy in tapping mode was performed using a Multimode 8 with Nanoscope 4 controller. An E or J scanner with maximum scan size of 10 and 100 µm was used for scanning samples. For SFM-QNM measurements, both cantilevers with spring constants of 2 N/m (OLTESPA) and 0.4 N/m (ScanAsyst Air-HR) were used. The sensitivity of cantilevers was first calibrated by thermal tuning process. Then their spring constant was measured over Sapphire (Bruker) as a hard surface. The force applied to the samples by the tips was within the range of 100-2000 pN. Measurements were carried out in 512 in 512 pixels resolution and speed of 6 - 15 minutes per image at room temperatures 25-27°C and relative humidity 30 - 35%. SFM images were processed and analyzed with SPIP (Image Metrology A/S) software. Topography images were line flattened with first order polynomial. Scanning electron microscopy (SEM): Scanning electron microscopy (SEM) images were obtained using an SEM machine from TESCAN Company, Brno, (Czech Republic) under vacuum at an operating voltage of 10 kV. Water dispersion of samples (0.1 mg/ml) were dropped on a grid and solvent was evaporated at room temperature. Dried samples were coated with a thin layer of gold by sputtering for 15 s and used for SEM imaging.

Transmission Electron Microscopy (TEM): Carbon-coated collodium film covered copper grids (400 mesh) were hydrophilized by a 60 s glow discharging at 8 W in a BALTEC MED 020 device (Leica Microsystems, Wetzlar, Germany). Then 5 µl of the PTrz dispersion in water (0,01 mg/ml) was absorbed onto the hydrophilized grids. The supernatant fluid was removed by blotting with a filter paper and the sample was allowed to dry in air. A standard holder was used to transfer the dried samples into a FEI Talos L120C transmission electron microscope (Thermo Fisher Scientific Inc., Waltham, Massachusetts, USA) equipped with a tungsten cathode. Images were taken with a 4k × 4k Ceta CMOS camera at a primary magnification of 36.000 × using an accelerating voltage of 120 kV.

Cryo-TEM: Perforated (1 µm hole diameter) carbon film-covered microscopical 200 mesh grids (R1/4 batch of Quantifoil, MicroTools GmbH, Jena, Germany) were hydrophilized by a 60 s glow discharging at 8 W in a BALTEC MED 020 device (Leica Microsystems, Wetzlar, Germany). Then 5 µl of PTrz18 dispersion in water (0.01 mg/ml) was pipetted to the hydrophilized grid and the supernatant fluid was immediately removed with a piece of filter paper until an ultrathin layer of the sample solution was obtained spanning the holes of the carbon film. The samples were vitrified by automatic blotting and plunge freezing with a FEI Vitrobot Mark IV (Thermo Fisher Scientific Inc., Waltham, Massachusetts, USA) using liquid ethane as cryogen. The vitrified specimens were transferred to the autoloader of a FEI TALOS ARCTICA electron microscope (Thermo Fisher Scientific Inc., Waltham, Massachusetts, USA). This microscope is equipped with a high-brightness field-emission gun (XFEG) operated at an acceleration voltage of 200 kV. Micrographs were acquired on a FEI Falcon 3 direct electron detector (Thermo Fisher Scientific Inc., Waltham, Massachusetts, USA) using a 100 µm objective aperture at a nominal magnification of 28,000 x, corresponding to a calibrated pixel size of 3.69 Å/pixel. Cryo-Electron Tomography (Cryo-ET): Tomograms were acquired on a TALOS ARCTICA transmission electron microscope (ThermoFisher Scientific Inc., Waltham (MA), USA) operating at 200 kV. Single axis tilt series (±64° in 2° tilt angle increments) were recorded with a FEI Falcon 3 4k × 4k direct electron detector at full resolution (29 K primary magnification). Tomogram reconstruction was done using ThermoFisher Inspect3D software, Version 3.1.0. The 3D voltex presentation was prepared using Amira, Version 6.2 (ThermoFisher Scientific Inc., Waltham (MA), USA).

Energy-dispersive X-ray spectroscopy (EDX): Energy-dispersive X-ray spectroscopy (EDX) images of the synthesized materials were obtained using SEM (TESCAN, Brno, Czech Republic) under vacuum at an operating voltage of 10 kV integrated with an energy- dispersive X-ray spectrometer (EDX).

Powder X-ray diffraction (P-XRD): P-XRD diffractograms of PTrz were recorded using a Siemens diffractometer with Cu-k radiation at 35 kV in the scan range of 2θ = 2° to 90°.

Raman spectroscopy: The Raman spectra of the synthesized materials were acquired with a Horiba XploRA spectrometer. The laser light of 532 nm (2.33 eV) was focused on the samples containing molecules and polymers with a 100x objective (N.A.=0.95). The backscattering signals were collected by the same lens. The elastic signal was removed by a long-pass filter and Raman light was dispersed by 1200 lines/mm grating and detected by a thermoelectrically cooled charge coupled devices (CCD) detector.

### Example 1: Reaction of sodium acetlyide and cyanuric choride (2,4,6-trichloro-1,3,5-trazine)

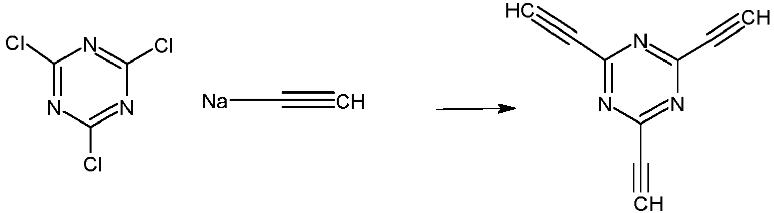

Cyanuric chloride (2g, 10.8 mmol) was dissolved in THF (30 ml) and sodium acetylide (1.5 g, 32 mmol), (9.96 ml in Xylol (18w/w%)) was stepwise (in 20 min) added to this solution. Solution was changed to yellow (after 15 min), red (after 30 min) and brawn (after 1 h) gradually. Solution was stirred for 2h, 6h and 18 h to obtain corresponding products which were abbreviated PTrz2h, PTrz6h and PTrz18h, respectively. Product of reactions were collected by centrifugation at 6000 rpm/20 °C for 30 min. The precipitate was dispersed in water and NMP alternatively and collected by centrifugation, at the same conditions. Finally, product was dispersed in water several times and collected by centrifugation. Reaction was performed under nitrogen.

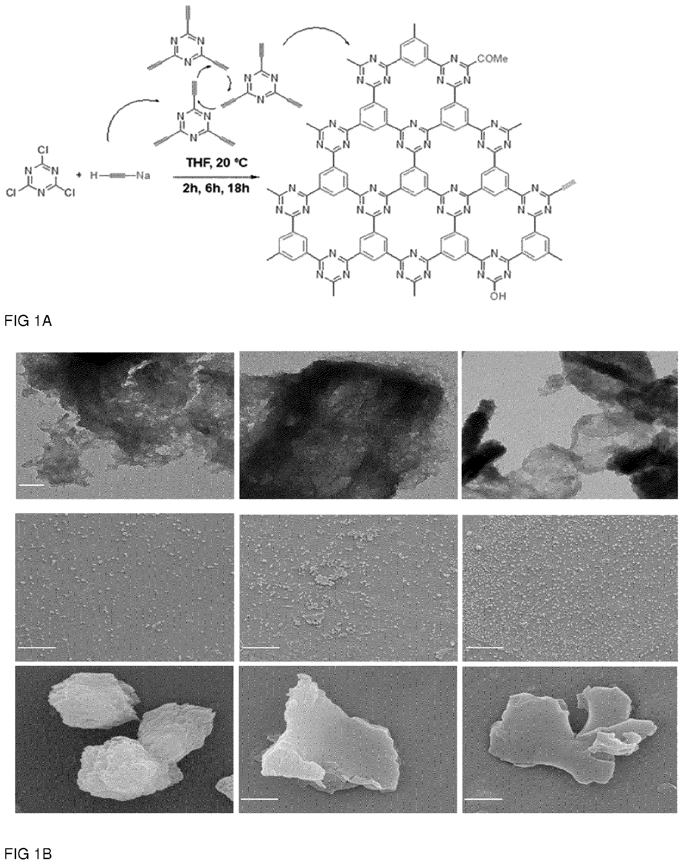

Fig. 1A shows the schematic representation of the synthesis of sheet-like polytriazine heteroaromatic structures. Nucleophilic substitution of chlorine atoms of cyanuric chloride by sodium acetylide resulted in reactive intermediates comprising a triazine core and ethynyl groups that changed to sheet-like polytriazine heteroaromatic structures (PTrz) upon cyclotrimerization). The growth of the sheets was monitored by performing the reaction at three different time frames. Products of 2h, 6h and 18 h reactions were labelled as PTrz2, PTrz6 and PTrz18, respectively. The product of the 2 hours reaction consisted of inhomogeneous flat materials laterally connected with bigger sheets. After six hours, sheet-like structures with clear edges in the micrometer range were formed. Small particles joining the edges of the big sheets indicated sheets with lateral growth. After 18 hours reaction, the product contained sheet-like structures with several micrometers lateral size and clear edges (Fig. 1B).

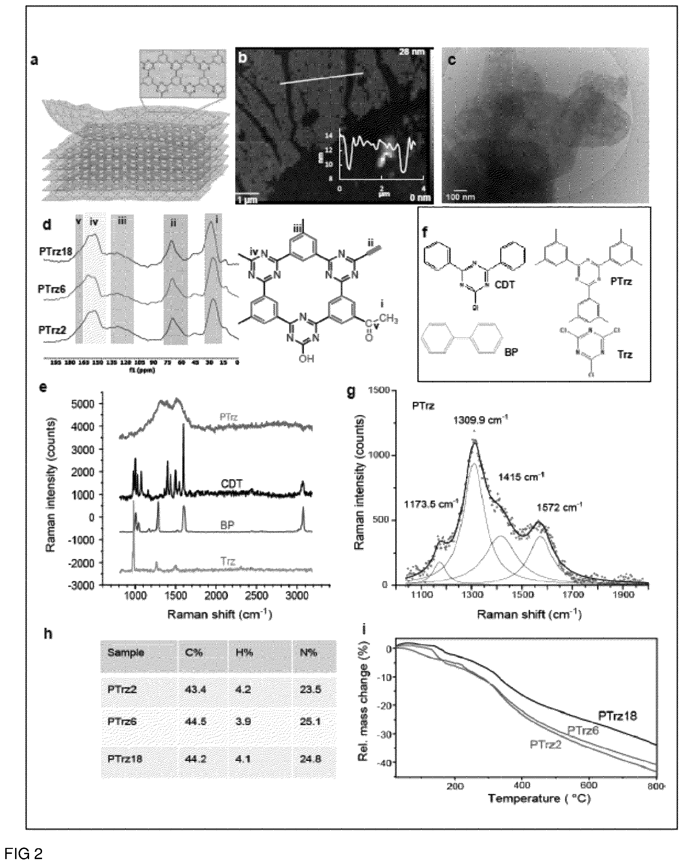

The thickness and basal plane of the materials increased simultaneously with increasing reaction time, pointing towards concurrent lateral and vertical growth. Lateral crosslinking and vertical growth of the materials correspond to the cyclotrimerization of ethynyl groups and π-π stacking of their backbone. In some cases, big holes with few hundred nanometers diameter and -30 nm depth on the basal plan of PTrz18 were also observed (Fig. 2A). These holes were assigned to the mismatched lateral crosslinking of small sheets during the formation of sublayer and driving the growth of upper layers to the same topology by π-π interactions.

The noncovalent interactions between the PTrz18 layers was confirmed by scotch tape exfoliation to few layers (Fig. 2AB). The AFM results were confirmed by TEM image that showed a clear trend from irregular materials towards big sheets as reaction time progressed (Figure 1B).While PTrz2 did not show any clear morphology, the product of the 18 hours reaction was containing integrated sheet-like structures with several micrometer lateral size and observable folds. Small particles attaching to the periphery of big cores conformed the lateral growth of PTrz (Figure

Thanks to the water dispersibility of PTrz18, the morphology of this compound was also investigated by Cryo-TEM and Cryo-electron tomography (cryo-ET) (Figure 2c). In agreement with the AFM and conventional TEM images, cryo-ET showed a sheet-like structure for this compound. Tomograms (not shown) revealed that PTrz18 consist of many stacked layers with -100 nm thickness. Accordingly, the SEM images showed a flat morphology for PTrz6 and PTrz18. The segregated structure of PTrz2 and unified structures of PTrz6 and PTrz18 indicated integrating of the backbone of these materials over time (Figure 1B).

The structure of PTrz was investigated by ¹³C solid-state cross-polarization magic-angle-spinning (CP-MAS) nuclear magnetic resonance (NMR) spectra (Figure 2d). The NMR spectra of PTrz2, PTrz6 and PTrz18 showed signals at 150-170 ppm and 100-150 ppm, which were assigned to the carbon atoms of triazine and benzene rings, respectively. The signal of benzene ring corresponds to the cyclotrimerization of ethynyl groups. The signal at 55-75 ppm was attributed to the unreacted ethynyl groups. The shoulder at 170-180 ppm together with the signal at 30 ppm were assigned to the methyl ketone functional groups (Figure 2d). The pathway of creation methyl ketone functional groups is discussed in the mechanism section. The similarity of the NMR spectra of PTrz2, PTrz6 and PTrz18 showed that the main backbone of these materials forms in a short time frame. Lengthening the reaction time develops the basal plane and improves the integration through lateral crosslinking. The IR spectra of the product of different reaction times showed absorbance bands at 1640-1650 cm-1 and 1520-1530 cm-1 for stretching vibrations of the carbon- carbon and carbon-nitrogen double bonds, respectively. A broad absorbance band at 2900-3600 cm-1 corresponds to the hydroxyl functional groups of PTrz (not shown). The Raman spectra of PTrz6 and PTrz18 showed two broad peaks centered at 1550 cm-1 and 1350 cm-1 and slightly shifted peaks at 1558 cm-1 and 1344 cm-1 for PTrz2. The peak at 1550 cm-1 showed 30 cm-1 downshift in comparison with G peak of graphite. This agrees with a shift induced by the heavier nitrogen atoms when assuming the frequency to be inversely proportional to the vibrating atoms. A Raman spectra was recorded of small molecules including 2,4,6-trichloro-1,3,5-triazine (Trz), biphenyl (BP), 2-chloro-4 6-diphenyl-1 3 5-triazine (CDT) with a structure similar to the repeating unit of PTrz were recorded (Figure 2e,f).

### Example 2: Reaction of sodium acetlyide and 2-chloro-4,6-diphenyl-1,3,5-triazine

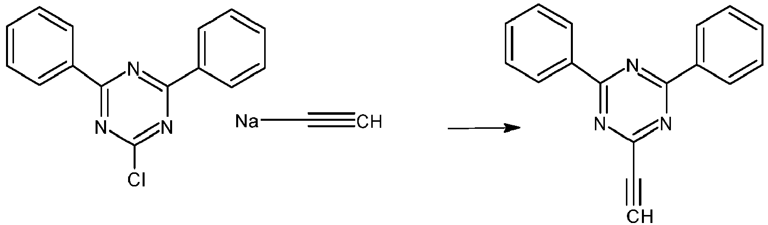

Sodium acetylide in xylene (18%w/w) (0.089 g, 1.8 mmol) (0.57 ml)) was added to a solution of 2-chloro-4,6-diphenyl-1,3,5-triazine (0.5 g, 1.8 mmol) in THF (15 ml) at room temperature and mixture was stirred under nitrogen at this temperature for 18 h. Solvent was evaporated and crude product was dispersed in dichloromethane and washed with water and the organic phase was separated by dropping funnel. Then solvent was evaporated and compound was dispersed in methanol and then filtered. Methanol was evaporated and the white solid compound was washed by pentane. Thin layer chromatography (TLC) in dichloromethane/pentane (1/5) showed two points. Top point was belonging to the precursor which was separated from the product by washing with pentane. The product was dissolved in DMSO and left in refrigerator to obtain crystals.

### Example 3: Reaction between sodium acetylide and 2-chloro-4,6-methoxy-1,3,5-triazine

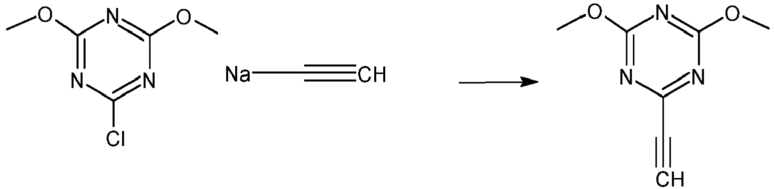

2-chloro-4,6-methoxy-1,3,5-triazine (0.5 g, 2.8 mmol) was dissolved in 10 ml THF and sodium acetylide (0.13 g, 2.8 mmol, 0.88 ml in xylol w/w%) was added to this solution and mixture was stirred at room temperature under nitrogen for 4 days. Solution was changed to yellow. The solvent including residue of xlylol was evaporated at 50 °C under vacuum and then crude product was dissolved in chloroform (3 ml) and left in refrigerator to remove the unreacted precursor as a white precipitate. Then it was filtered and the solvent was evaporated under vacuum. After 10 min at room temperature, yellow solid needle crystals were appeared. Extra high vacuum was needed to completely remove traces of xylol.

### Example 4: Reaction between cyanuric chloride, sodium acetylide and 2-chloro-4,6-diphenyl-1,3,5-triazine

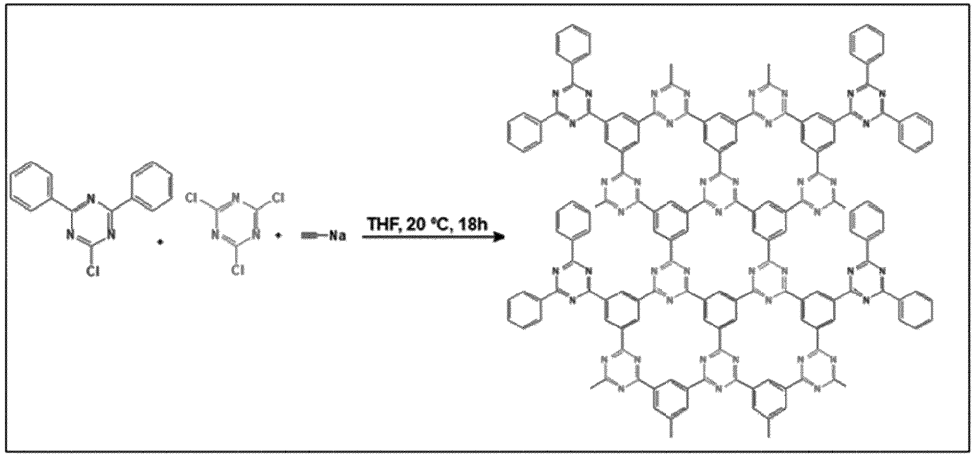

2-chloro-4,6-diphenyl-1,3,5-triazine (0.5 g, 1.8 mmol) and cyanuric chloride (0.82 g, 4.44 mmol) were dissolved in THF (20 ml) and stirred at room temperature for 30 min and sodium acetylide (0.729 g, 1.51 mmol) (4.6 ml in 18w/w% xlylol) was added to this solution at room temperature and mixture was stirred for 18 h under nitrogen. The color of solution was changed to orange and then black after several hours. Then precipitate was separated by centrifugation at 6000 rpm for 1 hour and supernatant was dialyzed in DMF and chloroform by dialysis bag cut off 50 kD for two weeks and two days, respectively. Solvent was evaporated and sample was collected for analysis.

### Example 5: Reaction between sodium acetylide and 2,4-dichloro-6-methoxy-1,3,5-triazine

2, 4-dichloro-6-methoxy-1,3,5-triazine (1.65 g, 9.19 mmol) was dissolved in 15 ml THF. Then sodium acetylide (0.88 g, 18.3 mmol, 5.6 ml in xlylol 18w/w%) was added to this solution and stirred under argon at room temperature for 48 hours. Solution was changed to orange in 30 min and then black in few hours. Water (10 ml) was slowly added to this mixture to denature excess of sodium acetylide. Then product was dialyzed against water for two weeks and then against methanol for two days by dialysis bag cut off 50 kD. Afterwards, solvent was evaporated and product was stored in water.

### Example 6: Intercalation of Rhodamine 6G and Gold nanoparticles (5 nm) by two-dimensional triazine networks

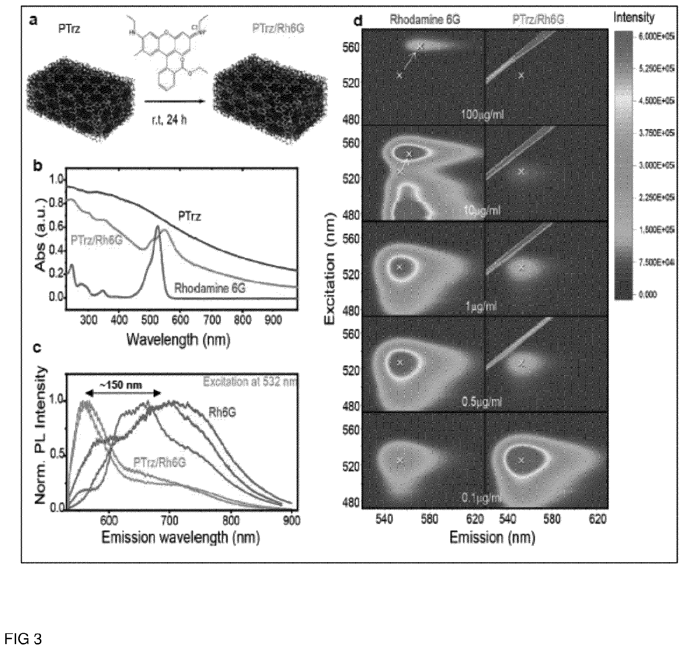

Intercalation of Rhodamine 6G_within in the sheet like material was tested (see Fig. 3a).

Water dispersion of PTrz-18 (1mg/ml) (5 ml) was sonicated for 5 min. Then Rh6G (10 mg) or gold nanoparticles (5 nm, 5 ml) were added to this dispersion and mixture was stirred for 30 min and then sonicated for 30 min. Mixture was then stirred for 2 h at room temperature and again sonicated for 30 min. Afterwards, mixture was stirred at room temperature for 18 h. Then, it was centrifuged at 6000 rpm/20 °C for 60 min, dispersed in water and collected by centrifugation at the same conditions.

PTrz18 showed 43% storage capacity for rhodamine 6g. The high capacity of PTrz18 for intercalation of rhodamine 6g was assigned to its porous structure and highly accessible surface area for dye molecules. The redshift in UV spectra of the intercalated rhodamine 6g (PTrz/rh) was due to the high local concentration of the dye and the aggregation inside the PTrz18 layers (Figure 3b). Intercalation of rhodamine 6g by PTrz18 gave rise to strong luminescence quenching. Figure 3c shows the luminescence of free and intercalated dye at various concentrations. At high concentration (100 µg/ml) neither free dye nor PTrz/rh showed any significant emission. Free dye displayed strong luminescence from 10-1 µg/ml concentrations. Strong quenching of the luminescence occurred after diluting down to 1 µg/ml. The local concentration dependent on-off emission of rhodamine 6g has been used to study the physicochemical properties of liposomes abundantly. The strong quenching of rhodamine 6g upon encapsulation by liposomes and raising its emission after releasing into the medium is an indicator to evaluate the pharmacokinetics of these systems. In contrast, a solution of PTrz/rh with only 0.1 µg/ml concentration showed strong emission. Considering 43% loading capacity, dye content of PTrz/rh at this concentration was 89 picomolar.

Free dye and PTrz/rh with 0.1 µg/ml concentration were dropped on silica, the solvent was evaporated at room temperature and then the samples were excited by laser 532 nm. Clear redshift, -150 nm, together with broadening of luminescence of free dye in comparison with PTrz/rh showed that dye aggregates upon evaporation of solvent. Intercalation of dye molecules, however, inhibited their aggregation during the evaporation of the solvent and PTrz/rh displayed an emission similar to the non-aggregated rhodamine 6g (Figure 3d). The maximum emission of intercalated dye at 560 nm with a shoulder at longer wavelengths was consistent with the PLE map of PTrz/rh in the solution state. Therefore, the observed intense luminescence for the PTrz/rh in solution was corresponding to the intercalated dye molecules.

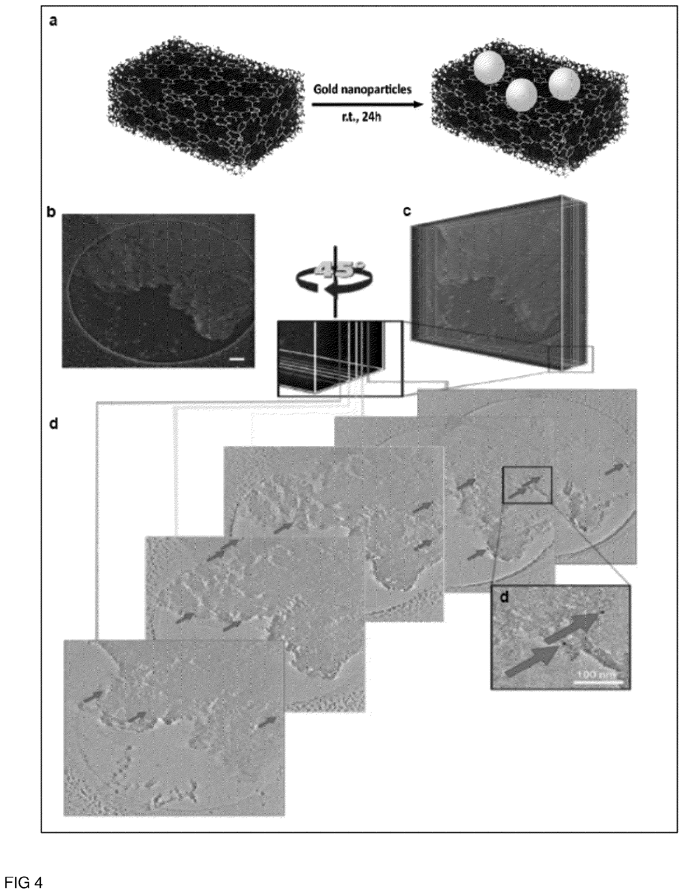

The ability of sheet-like benzene-triazine heteroaromatic structures for the intercalation of bigger objects was also investigated. Spherical gold nanoparticles with 5nm size were mixed with PTrz18, sonicated and stirred for several days at room temperature (Figure 4A). UV spectra did not show a clear indication for the encapsulation of gold nanoparticles by PTrz18 (not shown). To gain more information about the intercalation of gold nanoparticles, Cryo-ET was employed. According to the 3D reconstructed volume, only few gold nanoparticles penetrated the sheets, apparently only at the edges or through defects in the material (Figure 4B). In the defect-free basal plane of two- dimensional nanomaterials no intercalated gold nanoparticle were detected, highlighting the site- selective penetration of nanoscale particles. The ability of PTrz18 for intercalation of small molecules but blocking larger objects highlights their potential applications for new devices including membranes.

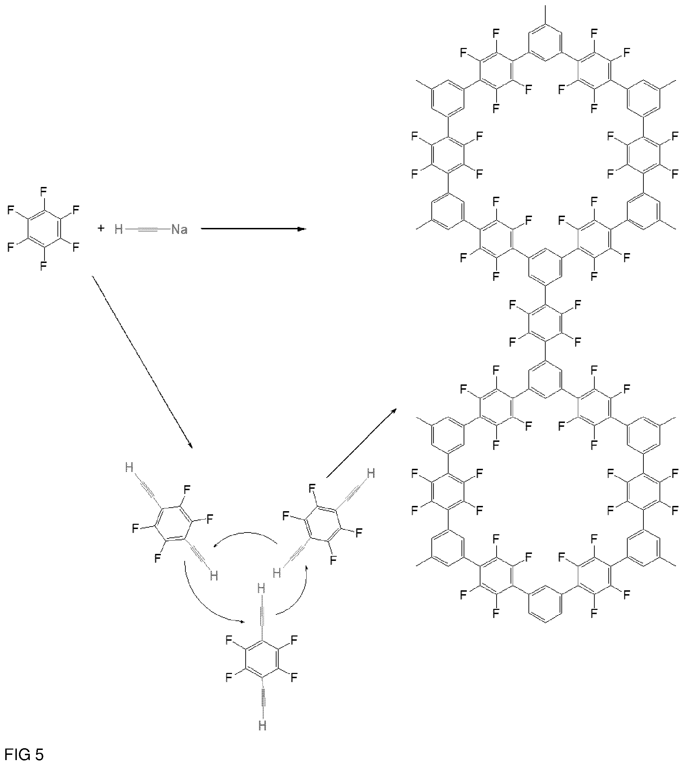

### Example 7: Reaction between hexafluorobenzene and sodium acetylide (See Fig. 5)

Hexafluorobenzene (2.97 g, 16 mmol) was dissolved in 1 4-dioxane (30 ml) and sodium acetylide (1.5 g, 32 mmol), (9.96 ml in Xylol (18w/w%)) was stepwise (in 20 min) added to this solution at room temperature. Mixture was stirred for 5 h at room temperature and then was refluxed for 4 days. Product of reactions were collected by centrifugation at 6000 rpm/20 °C for 30 min. The precipitate was dispersed in water, methanol and n-hexane alternatively and collected by centrifugation, at the same conditions. Finally product was dried at 50 °C for 4 h. Reaction was performed under nitrogen.

## Claims

1. A method for synthesizing a two-dimensional material consisting of conjugated aromatic structures by trimerization, which comprises reacting
- sodium acetylide orodium cyanide, , in particular sodium acetylide, and
- at least one electron deficient aromatic system of the general formulae (I) 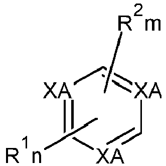
with R¹ being an electron withdrawing moiety selected from halogen, in particular -Cl or -F, -CF₃, -OCF₃, B(ORa)₂, SiRa₃, Si(ORa)₃ with Ra being H or alkyl, in particular C1-C10 alkyl
with n being 1-6,
with R² being selected from C1-C6 alkyl, C1-C6 alkoxy or substituted or unsubstituted C6 aryl moiety,
with m being 0-5,
with XA being independently from each other N, S, O or CH.

2. Method according to claim 1, **characterized in that**
R¹ being an electron withdrawing moiety selected from -Cl or-F, n being 1-6;
R² being selected from C1-C4 alkyl, such as -CH₃, -C2H₅, - C₃H₇, C₁-C₄ alkoxy, such as -OCH₃, -OC₂H₅ or -OC₃H₇, or phenyl, m being 0-5,
XA being independently from each other N or CH.

3. Method according to one of the preceding claims, **characterized by** at least one electron deficient aromatic system of the general formulae (IIa-c) 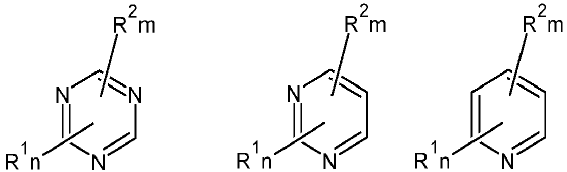
with R¹ being an electron withdrawing moiety selected from halogen, in particular -Cl or -F, -CF₃, -OCF₃,
With n being 1, 2, 3, 4 or 5, in particular 1, 2 or 3.
With R² being selected from C1-C4 alkyl, such as -CH₃, -C₂H₅, - C₃H₇, C₁-C₄ alkoxy, such as OCH₃, -OC₂H₅ or -OC₃H₇, or phenyl,
with m being 0, 1, 2, 3 or 4.

4. Method according to one of the preceding claims, **characterized by** at least one electron deficient aromatic system of the general formulae (IIIa-c) 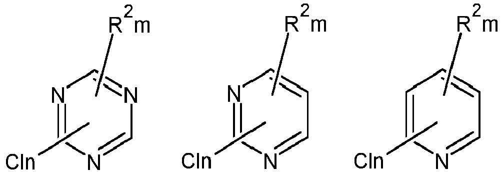
With n being 1, 2, 3, 4 or 5, in particular 1, 2 or 3,
With R² being selected from C1-C4 alkyl, such as -CH₃, -C2H₅, - C₃H₇, C₁-C₄ alkoxy, such as OCH₃, -OC₂H₅ or -OC₃H₇, or phenyl,
with m being 0, 1, 2, 3 or 4.

5. Method according to one of the preceding claims, **characterized by** at least one electron deficient aromatic system of the general formulae (IV) 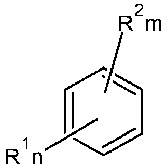
with R¹ being an electron withdrawing moiety selected from halogen, in particular -Cl or -F, -CF₃, -OCF₃,
with n being 1-6, in particular 4, 5 or 6,
with R² being selected from C1-C4 alkyl, such as -CH₃, -C₂H₅, - C₃H₇, C₁-C₄ alkoxy, such as OCH₃, -OC₂H₅ or -OC₃H₇, or phenyl,
with m being 0, 1, 2, 3 , 4 or 5, in particular 0, 1, 2.

6. Method according to one of the preceding claims, **characterized by** at least one electron deficient aromatic system of the general formulae (V) 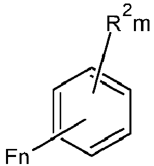
with n being 1-6, in particular 4, 5 or 6,
with R² being selected from C1-C4 alkyl, such as -CH₃, -C2H₅, - C₃H₇, C₁-C₄ alkoxy, such as OCH₃, -OC₂H₅ or -OC₃H₇, or phenyl,
with m being 0, 1, 2, 3 , 4 or 5, in particular 0, 1, 2.

7. Method according to one of the preceding claims **characterized in that** at least two of the electron deficient aromatic system of the general formulae (I) are reacted with sodium acetylide.

8. Method according to one of the preceding claims, **characterized in that** the reaction is carried in an organic solvent, in particular THF, at room temperature.

9. A two-dimensional material with conjugated aromatic structures obtainable in a method according to one of the preceding claims, comprising at least one of the following structure units: 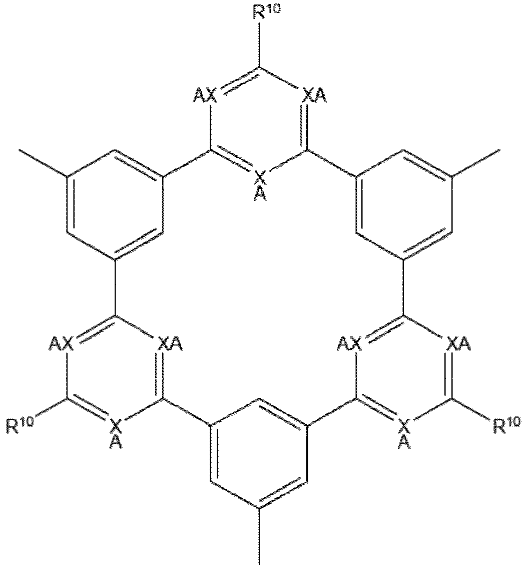
with XA being independently from each other N, S, O or CH, in particular N or CH;
with R¹⁰ being selected from -CHCH, -OH, C1-C6 alkyl, in particular C1-C4 alkyl, such as -CH₃, -C2H₅, - C₃H₇, C1-C6 alkoxy, in particular C₁-C₄ alkoxy, such as - OCH₃, -OC₂H₅ or -OC₃H₇, substituted or unsubstituted C6 aryl moiety , in particular phenyl.

10. A two-dimensional material with conjugated aromatic structures obtainable in a method according to one of claims 1-9, comprising at least one of the following structure units: 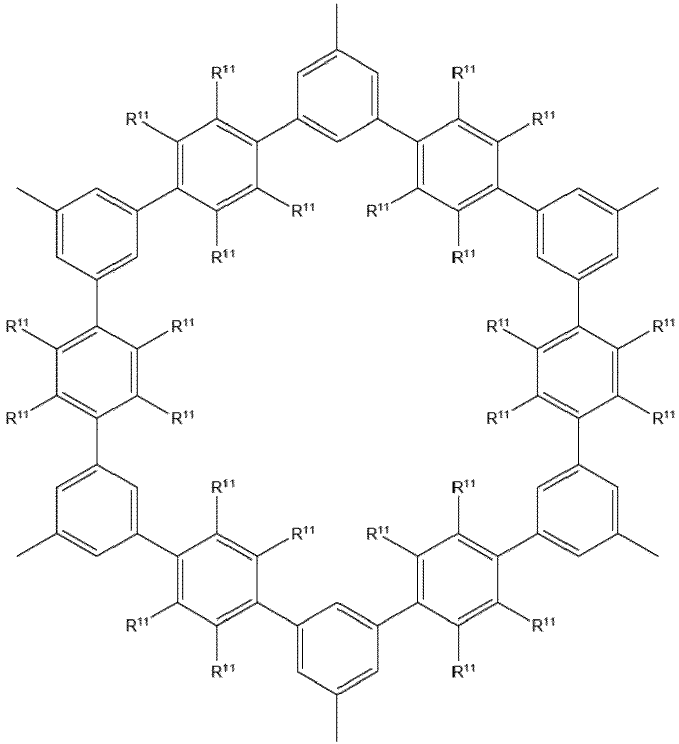 with R¹¹ being an electron withdrawing moiety selected from halogen, in particular -Cl or -F, -CF₃, -OCF₃, B(ORa)₂, SiRa₃, Si(ORa)₃ with Ra being H or alkyl, in particular C1-C10 alkyl.

11. Use of a two-dimensional material according to one of the claims 9 or 10 as filter material for small molecules or compounds, in particular molecules with a size larger 5 nm.
